# EUROPEAN PATENT APPLICATION

(11) **EP 3 252 077 A1**
(43) Date of publication of application: **06.12.2017**
(21) Application number: 17174088.9
(22) Date of filing: 15.07.2011
(51) Int. Cl.: C07K 16/28, A61K 39/395, A61P 35/02

(54) **SUPERIOR EFFICACY OF CD37 ANTIBODIES IN CLL BLOOD SAMPLES**

(30) Priority: 16.07.2010 EP 10169795; 07.09.2010 EP 10175586
(62) Divisional of application: 11732464.0
(71) Applicant: Boehringer Ingelheim International GmbH, 55216 Ingelheim am Rhein (DE)
(72) Inventor: STILGENBAUER, Stephan, 89081 Ulm (DE); ZENZ, Thorsten, 69120 Heidelberg (DE); HEIDER, Karl-Heinz, 55216 INGELHEIM AM RHEIN (DE)
(74) Representative: Simon, Elke Anna Maria

(57) **Abstract**

The present invention describes CD37 antibodies, especially A2 and B2, for the treatment of patients with CLL, especially of patients belonging to a "high risk" or "ultra-high risk" group of patients. Those patients are either patients who are refractory to fludarabine treatment or patients who carry a genetic marker which is indicative for poor prognosis or increased risk of treatment failure, e.g. patients with TP53 dysfunction or deletion of chromosome 17p13, or patients after failure to previous anti-CD20 treatment. The ability of A2 and B2 to deplete CLL cells is high both in patient samples derived from patients with normal risk and with increased risk ("high risk" patients) and clearly superior to that of rituximab and alemtuzumab.

SEQUENCE LISTING

<110>  Boehringer Ingelheim International GmbH

<120>  SUPERIOR EFFICACY OF CD37 ANTIBODIES IN CLL BLOOD SAMPLES

<130>  01-2642-EP-4 Div

<160>  21

<170>  PatentIn version 3.3

<210>  1
<211>  348
<212>  DNA
<213>  Mus musculus

<400>  1
gcggtccagc tgcagcagtc tggacctgag ctggaaaagc ctggcgcttc agtgaagatt      60

tcctgcaagg cttctggtta ctcattcact ggctacaata tgaactgggt gaagcagaat     120

aatggaaaga gccttgagtg gattggaaat attgatcctt attatggtgg tactacctac     180

aaccggaagt tcaagggcaa ggccacattg actgtagaca aatcctccag cacagcctac     240

atgcagctca agagtctgac atctgaggac tctgcagtct attactgtgc aagatcggtc     300

ggccctatgg actactgggg tcaaggaacc tcagtcaccg tctcttct                  348


<210>  2
<211>  116
<212>  PRT
<213>  Mus musculus

<400>  2

Ala Val Gln Leu Gln Gln Ser Gly Pro Glu Leu Glu Lys Pro Gly Ala
1               5                   10                  15


Ser Val Lys Ile Ser Cys Lys Ala Ser Gly Tyr Ser Phe Thr Gly Tyr
            20                  25                  30


Asn Met Asn Trp Val Lys Gln Asn Asn Gly Lys Ser Leu Glu Trp Ile
        35                  40                  45


Gly Asn Ile Asp Pro Tyr Tyr Gly Gly Thr Thr Tyr Asn Arg Lys Phe
    50                  55                  60


Lys Gly Lys Ala Thr Leu Thr Val Asp Lys Ser Ser Ser Thr Ala Tyr
65                  70                  75                  80


Met Gln Leu Lys Ser Leu Thr Ser Glu Asp Ser Ala Val Tyr Tyr Cys
                85                  90                  95


Ala Arg Ser Val Gly Pro Met Asp Tyr Trp Gly Gln Gly Thr Ser Val

            100                 105                 110

Thr Val Ser Ser
        115

<210> 3
<211> 324
<212> DNA
<213> Mus musculus

<400> 3
gacatccaga tgactcagtc tccagcctcc ctatctgcat ctgtgggaga gactgtcacc 60

atcacatgtc gaacaagtga aaatgtttac agttatttgg cttggtatca gcagaaacag 120

ggaaaatctc ctcagctcct ggtctctttt gcaaaaacct tagcagaagg tgtgccatca 180

aggttcagtg gcagtggatc aggcacacag ttttctctga agatcagcag cctgcagcct 240

gaagattctg gaagttattt ctgtcaacat cattccgata atccgtggac gttcggtgga 300

ggcaccgaac tggagatcaa acga 324

<210> 4
<211> 108
<212> PRT
<213> Mus musculus

<400> 4

Asp Ile Gln Met Thr Gln Ser Pro Ala Ser Leu Ser Ala Ser Val Gly
1               5                   10                  15

Glu Thr Val Thr Ile Thr Cys Arg Thr Ser Glu Asn Val Tyr Ser Tyr
            20                  25                  30

Leu Ala Trp Tyr Gln Gln Lys Gln Gly Lys Ser Pro Gln Leu Leu Val
        35                  40                  45

Ser Phe Ala Lys Thr Leu Ala Glu Gly Val Pro Ser Arg Phe Ser Gly
    50                  55                  60

Ser Gly Ser Gly Thr Gln Phe Ser Leu Lys Ile Ser Ser Leu Gln Pro
65                  70                  75                  80

Glu Asp Ser Gly Ser Tyr Phe Cys Gln His His Ser Asp Asn Pro Trp
                85                  90                  95

Thr Phe Gly Gly Gly Thr Glu Leu Glu Ile Lys Arg
            100                 105

<210> 5
<211> 446

<212>  PRT
<213>  Artificial

<220>
<223>  Synthetic construct heavy chain A2

<400>  5

Ala Val Gln Leu Gln Gln Ser Gly Pro Glu Leu Glu Lys Pro Gly Ala
1               5                   10                  15

Ser Val Lys Ile Ser Cys Lys Ala Ser Gly Tyr Ser Phe Thr Gly Tyr
            20                  25                  30

Asn Met Asn Trp Val Lys Gln Asn Asn Gly Lys Ser Leu Glu Trp Ile
        35                  40                  45

Gly Asn Ile Asp Pro Tyr Tyr Gly Gly Thr Thr Tyr Asn Arg Lys Phe
    50                  55                  60

Lys Gly Lys Ala Thr Leu Thr Val Asp Lys Ser Ser Ser Thr Ala Tyr
65                  70                  75                  80

Met Gln Leu Lys Ser Leu Thr Ser Glu Asp Ser Ala Val Tyr Tyr Cys
                85                  90                  95

Ala Arg Ser Val Gly Pro Met Asp Tyr Trp Gly Gln Gly Thr Ser Val
            100                 105                 110

Thr Val Ser Ser Ala Ser Thr Lys Gly Pro Ser Val Phe Pro Leu Ala
        115                 120                 125

Pro Ser Ser Lys Ser Thr Ser Gly Gly Thr Ala Ala Leu Gly Cys Leu
    130                 135                 140

Val Lys Asp Tyr Phe Pro Glu Pro Val Thr Val Ser Trp Asn Ser Gly
145                 150                 155                 160

Ala Leu Thr Ser Gly Val His Thr Phe Pro Ala Val Leu Gln Ser Ser
                165                 170                 175

Gly Leu Tyr Ser Leu Ser Ser Val Val Thr Val Pro Ser Ser Ser Leu
            180                 185                 190

Gly Thr Gln Thr Tyr Ile Cys Asn Val Asn His Lys Pro Ser Asn Thr
        195                 200                 205

Lys Val Asp Lys Arg Val Glu Pro Lys Ser Cys Asp Lys Thr His Thr
    210                 215                 220

Cys Pro Pro Cys Pro Ala Pro Glu Leu Leu Gly Gly Pro Asp Val Phe
225                 230                 235                 240

Leu Phe Pro Pro Lys Pro Lys Asp Thr Leu Met Ile Ser Arg Thr Pro
                245                 250                 255

Glu Val Thr Cys Val Val Val Asp Val Ser His Glu Asp Pro Glu Val
            260                 265                 270

Lys Phe Asn Trp Tyr Val Asp Gly Val Glu Val His Asn Ala Lys Thr
        275                 280                 285

Lys Pro Arg Glu Glu Gln Tyr Asn Ser Thr Tyr Arg Val Val Ser Val
    290                 295                 300

Leu Thr Val Leu His Gln Asp Trp Leu Asn Gly Lys Glu Tyr Lys Cys
305                 310                 315                 320

Lys Val Ser Asn Lys Ala Leu Pro Ala Pro Glu Glu Lys Thr Ile Ser
                325                 330                 335

Lys Ala Lys Gly Gln Pro Arg Glu Pro Gln Val Tyr Thr Leu Pro Pro
            340                 345                 350

Ser Arg Glu Glu Met Thr Lys Asn Gln Val Ser Leu Thr Cys Leu Val
        355                 360                 365

Lys Gly Phe Tyr Pro Ser Asp Ile Ala Val Glu Trp Glu Ser Asn Gly
    370                 375                 380

Gln Pro Glu Asn Asn Tyr Lys Thr Thr Pro Pro Val Leu Asp Ser Asp
385                 390                 395                 400

Gly Ser Phe Phe Leu Tyr Ser Lys Leu Thr Val Asp Lys Ser Arg Trp
                405                 410                 415

Gln Gln Gly Asn Val Phe Ser Cys Ser Val Met His Glu Ala Leu His
            420                 425                 430

Asn His Tyr Thr Gln Lys Ser Leu Ser Leu Ser Pro Gly Lys
        435                 440                 445

<210>  6
<211>  214
<212>  PRT
<213>  Artificial

<220>

<223>  Synthetic construct light chain A2

<400>  6

Asp Ile Gln Met Thr Gln Ser Pro Ala Ser Leu Ser Ala Ser Val Gly
1               5                   10                  15

Glu Thr Val Thr Ile Thr Cys Arg Thr Ser Glu Asn Val Tyr Ser Tyr
            20                  25                  30

Leu Ala Trp Tyr Gln Gln Lys Gln Gly Lys Ser Pro Gln Leu Leu Val
        35                  40                  45

Ser Phe Ala Lys Thr Leu Ala Glu Gly Val Pro Ser Arg Phe Ser Gly
    50                  55                  60

Ser Gly Ser Gly Thr Gln Phe Ser Leu Lys Ile Ser Ser Leu Gln Pro
65                  70                  75                  80

Glu Asp Ser Gly Ser Tyr Phe Cys Gln His His Ser Asp Asn Pro Trp
                85                  90                  95

Thr Phe Gly Gly Gly Thr Glu Leu Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  7
<211>  330
<212>  PRT

<213>  Artificial

<220>
<223>  Synthetic construct constant heavy chain

<400>  7

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330

<210>  8
<211>  106
<212>  PRT
<213>  Artificial

<220>
<223>  Synthetic construct constant light chain

<400>  8

Thr Val Ala Ala Pro Ser Val Phe Ile Phe Pro Pro Ser Asp Glu Gln
1               5                   10                  15

Leu Lys Ser Gly Thr Ala Ser Val Val Cys Leu Leu Asn Asn Phe Tyr
            20                  25                  30

Pro Arg Glu Ala Lys Val Gln Trp Lys Val Asp Asn Ala Leu Gln Ser
        35                  40                  45

Gly Asn Ser Gln Glu Ser Val Thr Glu Gln Asp Ser Lys Asp Ser Thr
    50                  55                  60

Tyr Ser Leu Ser Ser Thr Leu Thr Leu Ser Lys Ala Asp Tyr Glu Lys
65                  70                  75                  80

His Lys Val Tyr Ala Cys Glu Val Thr His Gln Gly Leu Ser Ser Pro
                85                  90                  95

Val Thr Lys Ser Phe Asn Arg Gly Glu Cys

100                 105

<210>  9
<211>  446
<212>  PRT
<213>  Artificial

<220>
<223>  Synthetic construct heavy chain A4

<400>  9

Ala Val Gln Leu Gln Gln Ser Gly Pro Glu Leu Glu Lys Pro Gly Ala
1               5                   10                  15

Ser Val Lys Ile Ser Cys Lys Ala Ser Gly Tyr Ser Phe Thr Gly Tyr
            20                  25                  30

Asn Met Asn Trp Val Lys Gln Asn Asn Gly Lys Ser Leu Glu Trp Ile
        35                  40                  45

Gly Asn Ile Asp Pro Tyr Tyr Gly Gly Thr Thr Tyr Asn Arg Lys Phe
    50                  55                  60

Lys Gly Lys Ala Thr Leu Thr Val Asp Lys Ser Ser Ser Thr Ala Tyr
65                  70                  75                  80

Met Gln Leu Lys Ser Leu Thr Ser Glu Asp Ser Ala Val Tyr Tyr Cys
                85                  90                  95

Ala Arg Ser Val Gly Pro Met Asp Tyr Trp Gly Gln Gly Thr Ser Val
            100                 105                 110

Thr Val Ser Ser Ala Ser Thr Lys Gly Pro Ser Val Phe Pro Leu Ala
        115                 120                 125

Pro Ser Ser Lys Ser Thr Ser Gly Gly Thr Ala Ala Leu Gly Cys Leu
    130                 135                 140

Val Lys Asp Tyr Phe Pro Glu Pro Val Thr Val Ser Trp Asn Ser Gly
145                 150                 155                 160

Ala Leu Thr Ser Gly Val His Thr Phe Pro Ala Val Leu Gln Ser Ser
                165                 170                 175

Gly Leu Tyr Ser Leu Ser Ser Val Val Thr Val Pro Ser Ser Ser Leu
            180                 185                 190

Gly Thr Gln Thr Tyr Ile Cys Asn Val Asn His Lys Pro Ser Asn Thr
        195                 200                 205

Lys Val Asp Lys Arg Val Glu Pro Lys Ser Cys Asp Lys Thr His Thr
    210                 215                 220

Cys Pro Pro Cys Pro Ala Pro Glu Leu Leu Ala Gly Pro Asp Val Phe
225                 230                 235                 240

Leu Phe Pro Pro Lys Pro Lys Asp Thr Leu Met Ile Ser Arg Thr Pro
                245                 250                 255

Glu Val Thr Cys Val Val Val Asp Val Ser His Glu Asp Pro Glu Val
            260                 265                 270

Lys Phe Asn Trp Tyr Val Asp Gly Val Glu Val His Asn Ala Lys Thr
        275                 280                 285

Lys Pro Arg Glu Glu Gln Tyr Asn Ser Thr Tyr Arg Val Val Ser Val
    290                 295                 300

Leu Thr Val Leu His Gln Asp Trp Leu Asn Gly Lys Glu Tyr Lys Cys
305                 310                 315                 320

Lys Val Ser Asn Lys Ala Leu Pro Ala Pro Glu Glu Lys Thr Ile Ser
                325                 330                 335

Lys Ala Lys Gly Gln Pro Arg Glu Pro Gln Val Tyr Thr Leu Pro Pro
            340                 345                 350

Ser Arg Glu Glu Met Thr Lys Asn Gln Val Ser Leu Thr Cys Leu Val
        355                 360                 365

Lys Gly Phe Tyr Pro Ser Asp Ile Ala Val Glu Trp Glu Ser Asn Gly
    370                 375                 380

Gln Pro Glu Asn Asn Tyr Lys Thr Thr Pro Pro Val Leu Asp Ser Asp
385                 390                 395                 400

Gly Ser Phe Phe Leu Tyr Ser Lys Leu Thr Val Asp Lys Ser Arg Trp
                405                 410                 415

Gln Gln Gly Asn Val Phe Ser Cys Ser Val Met His Glu Ala Leu His
            420                 425                 430

Asn His Tyr Thr Gln Lys Ser Leu Ser Leu Ser Pro Gly Lys
        435                 440                 445

<210>  10

<211>  214
<212>  PRT
<213>  Artificial

<220>
<223>  Synthetic construct light chain A4

<400>  10

Asp Ile Gln Met Thr Gln Ser Pro Ala Ser Leu Ser Ala Ser Val Gly
1               5                   10                  15

Glu Thr Val Thr Ile Thr Cys Arg Thr Ser Glu Asn Val Tyr Ser Tyr
            20                  25                  30

Leu Ala Trp Tyr Gln Gln Lys Gln Gly Lys Ser Pro Gln Leu Leu Val
        35                  40                  45

Ser Phe Ala Lys Thr Leu Ala Glu Gly Val Pro Ser Arg Phe Ser Gly
    50                  55                  60

Ser Gly Ser Gly Thr Gln Phe Ser Leu Lys Ile Ser Ser Leu Gln Pro
65                  70                  75                  80

Glu Asp Ser Gly Ser Tyr Phe Cys Gln His His Ser Asp Asn Pro Trp
                85                  90                  95

Thr Phe Gly Gly Gly Thr Glu Leu Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  11
<211>  446
<212>  PRT
<213>  Artificial

<220>
<223>  Synthetic construct heavy chain B2

<400>  11

Ala Val Gln Leu Val Gln Ser Gly Ala Glu Val Lys Lys Pro Gly Ser
1               5                   10                  15

Ser Val Lys Val Ser Cys Lys Ala Ser Gly Tyr Ser Phe Thr Gly Tyr
            20                  25                  30

Asn Met Asn Trp Val Arg Gln Ala Pro Gly Gln Gly Leu Glu Trp Met
        35                  40                  45

Gly Asn Ile Asp Pro Tyr Tyr Gly Gly Thr Thr Tyr Asn Arg Lys Phe
    50                  55                  60

Lys Gly Arg Val Thr Leu Thr Val Asp Lys Ser Ser Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Ser Val Gly Pro Met Asp Tyr Trp Gly Gln Gly Thr Leu Val
            100                 105                 110

Thr Val Ser Ser Ala Ser Thr Lys Gly Pro Ser Val Phe Pro Leu Ala
        115                 120                 125

Pro Ser Ser Lys Ser Thr Ser Gly Gly Thr Ala Ala Leu Gly Cys Leu
    130                 135                 140

Val Lys Asp Tyr Phe Pro Glu Pro Val Thr Val Ser Trp Asn Ser Gly
145                 150                 155                 160

Ala Leu Thr Ser Gly Val His Thr Phe Pro Ala Val Leu Gln Ser Ser
                165                 170                 175

Gly Leu Tyr Ser Leu Ser Ser Val Val Thr Val Pro Ser Ser Ser Leu
            180                 185                 190

Gly Thr Gln Thr Tyr Ile Cys Asn Val Asn His Lys Pro Ser Asn Thr
        195                 200                 205

Lys Val Asp Lys Arg Val Glu Pro Lys Ser Cys Asp Lys Thr His Thr
    210                 215                 220

Cys Pro Pro Cys Pro Ala Pro Glu Leu Leu Gly Gly Pro Asp Val Phe
225                 230                 235                 240

Leu Phe Pro Pro Lys Pro Lys Asp Thr Leu Met Ile Ser Arg Thr Pro
                245                 250                 255

Glu Val Thr Cys Val Val Val Asp Val Ser His Glu Asp Pro Glu Val
            260                 265                 270

Lys Phe Asn Trp Tyr Val Asp Gly Val Glu Val His Asn Ala Lys Thr
        275                 280                 285

Lys Pro Arg Glu Glu Gln Tyr Asn Ser Thr Tyr Arg Val Val Ser Val
    290                 295                 300

Leu Thr Val Leu His Gln Asp Trp Leu Asn Gly Lys Glu Tyr Lys Cys
305                 310                 315                 320

Lys Val Ser Asn Lys Ala Leu Pro Ala Pro Glu Glu Lys Thr Ile Ser
                325                 330                 335

Lys Ala Lys Gly Gln Pro Arg Glu Pro Gln Val Tyr Thr Leu Pro Pro
            340                 345                 350

Ser Arg Glu Glu Met Thr Lys Asn Gln Val Ser Leu Thr Cys Leu Val
        355                 360                 365

Lys Gly Phe Tyr Pro Ser Asp Ile Ala Val Glu Trp Glu Ser Asn Gly
    370                 375                 380

Gln Pro Glu Asn Asn Tyr Lys Thr Thr Pro Pro Val Leu Asp Ser Asp
385                 390                 395                 400

Gly Ser Phe Phe Leu Tyr Ser Lys Leu Thr Val Asp Lys Ser Arg Trp
                405                 410                 415

Gln Gln Gly Asn Val Phe Ser Cys Ser Val Met His Glu Ala Leu His
            420                 425                 430

Asn His Tyr Thr Gln Lys Ser Leu Ser Leu Ser Pro Gly Lys
        435                 440                 445

<210>  12
<211>  214

<212>  PRT
<213>  Artificial

<220>
<223>  Synthetic construct light chain B2

<400>  12

Asp Ile Gln Met Thr Gln Ser Pro Ser Ser Leu Ser Ala Ser Val Gly
1               5                   10                  15

Asp Arg Val Thr Ile Thr Cys Arg Thr Ser Glu Asn Val Tyr Ser Tyr
            20                  25                  30

Leu Ala Trp Tyr Gln Gln Lys Pro Gly Lys Ala Pro Lys Leu Leu Val
        35                  40                  45

Ser Phe Ala Lys Thr Leu Ala Glu Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Phe Cys Gln His His Ser Asp Asn Pro Trp
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  13
<211>  446
<212>  PRT
<213>  Artificial

<220>
<223>  Synthetic construct heavy chain B4

<400>  13

Ala Val Gln Leu Val Gln Ser Gly Ala Glu Val Lys Lys Pro Gly Ser
1               5                   10                  15

Ser Val Lys Val Ser Cys Lys Ala Ser Gly Tyr Ser Phe Thr Gly Tyr
            20                  25                  30

Asn Met Asn Trp Val Arg Gln Ala Pro Gly Gln Gly Leu Glu Trp Met
        35                  40                  45

Gly Asn Ile Asp Pro Tyr Tyr Gly Gly Thr Thr Tyr Asn Arg Lys Phe
    50                  55                  60

Lys Gly Arg Val Thr Leu Thr Val Asp Lys Ser Ser Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Ser Val Gly Pro Met Asp Tyr Trp Gly Gln Gly Thr Leu Val
            100                 105                 110

Thr Val Ser Ser Ala Ser Thr Lys Gly Pro Ser Val Phe Pro Leu Ala
        115                 120                 125

Pro Ser Ser Lys Ser Thr Ser Gly Gly Thr Ala Ala Leu Gly Cys Leu
    130                 135                 140

Val Lys Asp Tyr Phe Pro Glu Pro Val Thr Val Ser Trp Asn Ser Gly
145                 150                 155                 160

Ala Leu Thr Ser Gly Val His Thr Phe Pro Ala Val Leu Gln Ser Ser
                165                 170                 175

Gly Leu Tyr Ser Leu Ser Ser Val Val Thr Val Pro Ser Ser Ser Leu
            180                 185                 190

Gly Thr Gln Thr Tyr Ile Cys Asn Val Asn His Lys Pro Ser Asn Thr
        195                 200                 205

Lys Val Asp Lys Arg Val Glu Pro Lys Ser Cys Asp Lys Thr His Thr
    210                 215                 220

Cys Pro Pro Cys Pro Ala Pro Glu Leu Leu Ala Gly Pro Asp Val Phe
225                 230                 235                 240

Leu Phe Pro Pro Lys Pro Lys Asp Thr Leu Met Ile Ser Arg Thr Pro
                245                 250                 255

Glu Val Thr Cys Val Val Val Asp Val Ser His Glu Asp Pro Glu Val
            260                 265                 270

Lys Phe Asn Trp Tyr Val Asp Gly Val Glu Val His Asn Ala Lys Thr
        275                 280                 285

Lys Pro Arg Glu Glu Gln Tyr Asn Ser Thr Tyr Arg Val Val Ser Val
    290                 295                 300

Leu Thr Val Leu His Gln Asp Trp Leu Asn Gly Lys Glu Tyr Lys Cys
305                 310                 315                 320

Lys Val Ser Asn Lys Ala Leu Pro Ala Pro Glu Glu Lys Thr Ile Ser
                325                 330                 335

Lys Ala Lys Gly Gln Pro Arg Glu Pro Gln Val Tyr Thr Leu Pro Pro
            340                 345                 350

Ser Arg Glu Glu Met Thr Lys Asn Gln Val Ser Leu Thr Cys Leu Val
        355                 360                 365

Lys Gly Phe Tyr Pro Ser Asp Ile Ala Val Glu Trp Glu Ser Asn Gly
    370                 375                 380

Gln Pro Glu Asn Asn Tyr Lys Thr Thr Pro Pro Val Leu Asp Ser Asp
385                 390                 395                 400

Gly Ser Phe Phe Leu Tyr Ser Lys Leu Thr Val Asp Lys Ser Arg Trp
                405                 410                 415

Gln Gln Gly Asn Val Phe Ser Cys Ser Val Met His Glu Ala Leu His
            420                 425                 430

Asn His Tyr Thr Gln Lys Ser Leu Ser Leu Ser Pro Gly Lys
        435                 440                 445

<210>  14
<211>  214
<212>  PRT

<213>  Artificial

<220>
<223>  Synthetic construct light chain B4

<400>  14

Asp Ile Gln Met Thr Gln Ser Pro Ser Ser Leu Ser Ala Ser Val Gly
1               5                   10                  15

Asp Arg Val Thr Ile Thr Cys Arg Thr Ser Glu Asn Val Tyr Ser Tyr
            20                  25                  30

Leu Ala Trp Tyr Gln Gln Lys Pro Gly Lys Ala Pro Lys Leu Leu Val
        35                  40                  45

Ser Phe Ala Lys Thr Leu Ala Glu Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Phe Cys Gln His His Ser Asp Asn Pro Trp
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  15
<211>  5
<212>  PRT
<213>  Artificial

<220>
<223>  CDR1 heavy chain (H1)

<400>  15

Gly Tyr Asn Met Asn
1               5


<210>  16
<211>  17
<212>  PRT
<213>  Artificial

<220>
<223>  CDR2 heavy chain (H2)

<400>  16

Asn Ile Asp Pro Tyr Tyr Gly Gly Thr Thr Tyr Asn Arg Lys Phe Lys
1               5                   10                  15


Gly



<210>  17
<211>  7
<212>  PRT
<213>  Artificial

<220>
<223>  CDR3 heavy chain (H3)

<400>  17

Ser Val Gly Pro Met Asp Tyr
1               5


<210>  18
<211>  11
<212>  PRT
<213>  Artificial

<220>
<223>  CDR1 light chain (L1)

<400>  18

Arg Thr Ser Glu Asn Val Tyr Ser Tyr Leu Ala
1               5                   10


<210>  19
<211>  7
<212>  PRT

<213>  Artificial

<220>
<223>  CDR2 light chain (L2)

<400>  19

Phe Ala Lys Thr Leu Ala Glu
1               5


<210>  20
<211>  9
<212>  PRT
<213>  Artificial

<220>
<223>  CDR3 light chain (L3)

<400>  20

Gln His His Ser Asp Asn Pro Trp Thr
1               5


<210>  21
<211>  13
<212>  PRT
<213>  Artificial

<220>
<223>  alternative CDR2 heavy chain (H2b)

<400>  21

Asn Ile Asp Pro Tyr Tyr Gly Gly Thr Thr Tyr Asn Arg
1               5                   10

## Description

### BACKGROUND OF THE INVENTION

### TECHNICAL FIELD

The present invention relates to immunotherapies that are based on B cell depletion. In particular, the present invention relates to CD37 antibody molecules, especially A2 and B2, for use in such therapies, e.g. in the treatment of B cell malignancies and autoimmune conditions.

### BACKGROUND

Immunotherapy using monoclonal antibodies (mAbs) has been emerging as a safe and selective method for the treatment of cancer and other diseases. In particular, the role of monoclonal antibodies in therapies that are based on B cell depletion, e.g. in the treatment of B cell malignancies, has expanded since the introduction of rituximab (Rituxan®), an antibody that is directed against the CD20 antigen on the B cell surface. Numerous studies have confirmed the efficacy of rituximab as a single agent and in combination therapy in low-grade NHL (Hiddemann W, et al. Frontline therapy with rituximab added to the combination of cyclophosphamide, doxorubicin, vincristine, and prednisone (CHOP) significantly improves the outcome for patients with advanced-stage follicular lymphoma compared with therapy with CHOP alone: results of a prospective randomized study of the German Low-Grade Lymphoma Study. Group. Blood 2005; 106: 3725-3732 (2005)), mantle cell lymphoma (Forstpointner R, et al. The addition of rituximab to a combination of fludarabine, cyclophosphamide, mitoxantrone (FCM) significantly increases the response rate and prolongs survival as compared with FCM alone in patients with relapsed and refractory follicular and mantle cell lymphomas: results of a prospective randomized study of the German Low-Grade Lymphoma Study Group. Blood, 2004; 104: 3064-3071.), diffuse large B cell lymphoma (DLBCL) (Coiffier B, et al. Rituximab (anti-CD20 monoclonal antibody) for the treatment of patients with relapsing or refractory aggressive lymphoma: a multicenter phase II study. Blood 1998; 92: 1927-1932), and Burkitt lymphoma (Thomas DA, et al. Chemoimmunotherapy with hyper-CVAD plus rituximab for the treatment of adult Burkitt and Burkitt-type lymphoma or acute lymphoblastic leukemia. Cancer 2006; 106: 1569-1580).

However, only a subset of patients responds to therapy and the majority of those eventually relapse following rituximab treatment. Therefore, there is a need to find immunotherapies with higher efficacy than rituximab, especially with higher efficacy for those subsets of patients that respond poorly to rituximab treatment.

### SUMMARY OF THE INVENTION

The present invention describes CD37 antibodies, especially A2 and B2, for the treatment of patients with CLL, especially of patients belonging to a "high risk" group of patients. Those patients are either patients who are refractory to fludarabine treatment or patients who carry a genetic marker which is indicative for poor prognosis or increased risk of treatment failure, e.g. patients with TP53 mutation or deletion of chromosome 17p13. Antibodies A2 and B2 are tested in whole blood samples from CLL patients with respect to their ability to deplete malignant CLL cells. Rituximab, a CD20 antibody and alemtuzumab, a CD52 antibody are tested in parallel as comparator antibodies. Both A2 and B2 mediate a high degree of CLL cell depletion in these samples which is clearly superior to that of the CD20 antibody rituximab and the CD52 antibody alemtuzumab. A series of 21 blood samples derived from different patients is characterized according to their genetic status and response to fludarabine treatment (after clinical exposure and *in vitro* testing). Subset analysis of these patients reveals that the superior CLL depleting activity of A2 and B2 is present in all subsets analyzed. In particular, A2 and B2 are superior to rituximab and alemtuzumab in "high risk" patients that carry a deletion in chromosome 17p13, a TP53 mutation or are refractory to fludarabine treatment. Furthermore the CLL depleting effect of A2 and B2 is outstanding in normal risk patients as well as in "high risk" patients, indicating that the superior CLL depleting activity of CD37 antibodies, especially A2 and B2, is not restricted to patients with normal risk for treatment failure but applies particularly to patients bearing an increased risk for treatment failure. In contrast to the standard treatment CD37 antibodies, especially A2 and B2 show excellent CLL depleting activity across different CLL patient subgroups *in vitro* which is surprisingly clearly superior to approved antibodies rituximab and alemtuzumab.

Up to this point the only experimental data available for targeting CD37 on primary CLL cells *in vitro* indicate that CD37 antibodies or an anti-CD37 SMIP molecule are able to induce apoptosis and ADCC on primary CLL cells, however there is no evidence that CLL cell depletion in whole blood assays *in vitro* is observed (Zhao XB, Lapalombella R, Joshi T, Cheney C, Gowda A, Hayden-Ledbetter MS, Baum PR, Lin TS, Jarjoura D, Lehman A, Kussewitt D, Lee RJ, Caligiuri MA, Tridandapani S, Muthusamy N, Byrd JC. Targeting CD37+ lymphoid malignancies with a novel engineered small modular immunopharmaceutical 2007; BLOOD, 1 OCTOBER 2007, VOLUME 110, NUMBER 7, Epub ahead of print. Blood. 2007 Apr 17). Furthermore, there is no evidence that CLL cell depletion in patient derived blood samples using a CD37 antibody is superior to that of using other B cell directed antibodies. In recent publications by Zenz et al. (Abstract 2379, ASH 2009, New Orleans) and Platz et al. (Abstract 2365, ASH 2009, New Orleans) the efficacy of rituximab and the Fc-engineered novel CD20 antibody GA101 is tested in blood samples from CLL patients in a comparable assay format. The results disclosed in these presentations indicate that the novel Fc-engineered CD20 antibody GA101 is superior to rituximab, however the maximum observed effect of GA101 is clearly lower than that observed with A2 and B2. Hence, it is unexpected that an Fc-engineered CD37 antibody shows an activity that is even superior to that of an Fc-engineered CD20 antibody.

### ADVANTAGES

A high degree of CLL cell depletion in patients with CLL is considered advantageous for the treatment of CLL patients and is considered to translate into increased clinical benefit for patients treated with such an agent. Antibodies A2 and B2 display such a high degree of CLL cell depletion in whole blood assays which is superior to the effect of the CD20 antibodies rituximab (see data disclosed in this application) and GA101 (Zenz et al., 2009, Abstract 2379, ASH 2009, New Orleans). This superior efficacy of A2 and B2 is especially evident in patient samples which are derived from patients belonging to high risk groups, e.g. patients who are refractory to fludarabine or who carry genetic markers (e.g. TP53 mutation or 17p13 deletion) which are predictive for poor treatment outcome using current standard therapies (e.g. therapies combining a CD20 antibody with a fludarabine containing chemotherapy regimen). In addition, patients who failed previous anti-CD20 treatment are in need for improved treatment modalities. Therefore application of a CD20 unrelated treatment modality (e.g. antibodies specific for CD37, especially A2 and B2) is expected to provide an improved treatment for patients suffering from CLL, especially patients who belong to a high risk group of patients. The ability of CD37 antibodies, especially A2 and B2, to deplete CLL cells is high both in patient samples derived from patients with normal risk and with increased risk ("high risk" or "ultra-high risk" patients) and is clearly superior to that of rituximab and alemtuzumab.

### APPLICABILITY AND METHODS OF THERAPEUTIC USE

In accordance with the invention, there are provided novel indications of using CD37 antibodies as described in the present invention. Accordingly, the CD37 antibodies of the present invention may be used to treat "high risk" or "ultra high risk" patients suffering from B cell malignancies. To be used in therapy, the CD37 antibody is included into pharmaceutical compositions appropriate to facilitate administration to animals or humans. Typical formulations of the CD37 antibody molecule can be prepared by mixing the CD37 antibody molecule with physiologically acceptable carriers, excipients or stabilizers, in the form of lyophilized or otherwise dried formulations or aqueous solutions or aqueous or non-aqueous suspensions.
Pharmaceutically acceptable carriers and adjuvants for use with CD37 antibodies according to the present invention include, for example, ion exchangers, alumina, aluminum stearate, lecithin, serum proteins, buffer substances, water, salts or electrolytes and cellulose-based substances.

Carriers, excipients, modifiers or stabilizers are nontoxic at the dosages and concentrations employed. They include buffer systems such as phosphate, citrate, acetate and other anorganic or organic acids and their salts; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); proteins, such as serum albumin, gelatin, or immunoglobulins; hydrophilic polymers such as polyvinylpyrrolidone or polyethylene glycol (PEG); amino acids such as glycine, glutamine, asparagine, histidine, arginine, or lysine; monosaccharides, disaccharides, oligosaccharides or polysaccharides and other carbohydrates including glucose, mannose, sucrose, trehalose, dextrins or dextrans; chelating agents such as EDTA; sugar alcohols such as, mannitol or sorbitol; salt-forming counter-ions such as sodium; metal complexes (e.g. Zn-protein complexes); and/or ionic or non-ionic surfactants such as TWEEN™ (polysorbates), PLURONICS™ or fatty acid esters, fatty acid ethers or sugar esters. Also organic solvents can be contained in the antibody formulation such as ethanol or isopropanol. The excipients may also have a release-modifying or absorption-modifying function. This is not a complete list of possible pharmaceutically acceptable carriers and adjuvants, and one of ordinary skilled in the art would know other possibilities, which are replete in the art.

The CD37 antibody molecules may also be dried (freeze-dried, spray-dried, spray-freeze dried, dried by near or supercritical gases, vacuum dried, air-dried), precipitated or crystallized or entrapped in microcapsules that are prepared, for example, by coacervation techniques or by interfacial polymerization using, for example, hydroxymethylcellulose or gelatin and poly-(methylmethacylate), respectively, in colloidal drug delivery systems (for example, liposomes, albumin microspheres, microemulsions, nano-particles and nanocapsules), in macroemulsions or precipitated or immobilized onto carriers or surfaces, for example by pcmc technology (protein coated microcrystals). Such techniques are known in the art.

Naturally, the pharmaceutical compositions / formulations to be used for *in vivo* administration must be sterile; sterilization may be accomplished be conventional techniques, e.g. by filtration through sterile filtration membranes.

It may be useful to increase the concentration of the CD37 antibody to come to a so-called high concentration liquid formulation (HCLF); various ways to generate such HCLFs have been described.

The CD37 antibody molecule may also be contained in a sustained-release preparation. Such preparations include solid, semi-solid or liquid matrices of hydrophobic or hydrophilic polymers, and may be in the form of shaped articles, e.g. films, sticks or microcapsules and may be applied via an application device. Examples of sustained-release matrices include polyesters, hydrogels (for example, poly(2-hydroxyethyl-methacrylate) or sucrose acetate butyrate), or poly(vinylalcohol)), polylactides (US 3,773,919), copolymers of L-glutamic acid and y ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated antibodies remain in the body for a long time, they may denature or aggregate as a result of exposure to moisture at 37°C, resulting in a loss of biological activity and possible changes in immunogenicity. Rational strategies can be devised for stabilization depending on the mechanism involved. For example, if the aggregation mechanism is discovered to be intermolecular S-S bond formation through thio-disulfide interchange, stabilization may be achieved by modifying sulfhydryl residues, lyophilization from acidic solutions, controlling moisture content, using appropriate additives, and developing specific polymer matrix compositions.

The CD37 antibody molecule, especially A2 and B2, can be incorporated also in other application forms, such as dispersions, suspensions or liposomes, tablets, capsules, powders, sprays, transdermal or intradermal patches or creams with or without permeation enhancing devices, wafers, nasal, buccal or pulmonary formulations, or may be produced by implanted cells or - after gene therapy - by the individual's own cells.

A CD37 antibody molecule, especially A2 and B2, may also be derivatized with a chemical group such as polyethylene glycol (PEG), a methyl or ethyl group, or a carbohydrate group. These groups may be useful to improve the biological characteristics of the antibody, e.g. to increase serum half-life or to increase tissue binding.

The preferred mode of application is parenteral, by infusion or injection (intravenous, intramuscular, subcutaneous, intraperitoneal, intradermal), but other modes of application such as by inhalation, transdermal, intranasal, buccal, oral, may also be applicable.

For therapeutic use, the compounds may be administered in a therapeutically effective amount in any conventional dosage form in any conventional manner. Routes of administration include, but are not limited to, intravenously, intramuscularly, subcutaneously, intrasynovially, by infusion, sublingually, transdermally, orally, topically or by inhalation, tablet, capsule, caplet, liquid, solution, suspension, emulsion, lozenges, syrup, reconstitutable powder, granule, suppository and transdermal patch. Methods for preparing such dosage forms are known (see, for example, H.C. Ansel and N.G. Popovish, Pharmaceutical Dosage Forms and Drug Delivery Systems, 5th ed., Lea and Febiger (1990)). A therapeutically effective amount can be determined by a skilled artisan based upon such factors as weight, metabolism, and severity of the affliction etc.
Preferably the active compound is dosed at about 1 mg to about 500 mg per kilogram of body weight on a daily basis. More preferably the active compound is dosed at about 1 mg to about 100 mg per kilogram of body weight on a daily basis.

For the prevention or treatment of disease, the appropriate dosage of antibody will depend on the type of disease to be treated, the severity and course of the disease, whether the antibody is administered for preventive or therapeutic purposes, previous therapy, the patient's clinical history and response to the antibody, and the discretion of the attending physician. The antibody is suitably administered to the patient at one time or over a series of treatments.

Depending on the type and severity of the disease, about 0.01 µg/kg to 40 mg/kg (e.g. 0.1 - 20 mg/kg) of antibody, especially of A2 and B2, is an initial candidate dosage for administration to the patient, whether, for example, by one or more separate administrations, or by continuous infusion. For repeated administrations over several days or longer, depending on the condition, the treatment is sustained until a desired suppression of disease symptoms occurs. However, other dosage regimens may be useful. The progress of this therapy is easily monitored by conventional techniques and assays, e.g. by determining the extent of B cell depletion (e.g. using flow cytometry).
For A2, the estimated weekly dose for a 70 kg human is in the range of 86 to 184 mg. The estimated human weekly dose for B2 for a 70 kg human is 189 to 404 mg.

The "therapeutically effective amount" of the antibody to be administered is the minimum amount necessary to prevent, ameliorate, or treat a disease or disorder.

B cell malignancies include, without limitation, B cell lymphomas (e.g. various forms of Hodgkin's disease, B cell non-Hodgkin's lymphoma (NHL) and related lymphomas (e.g. Waldenström's macroglobulinaemia (also called lymphoplasmacytic lymphoma or immunocytoma) or central nervous system lymphomas), leukemias (e.g. acute lymphoblastic leukemia (ALL), chronic lymphocytic leukemia (CLL; also termed B cell chronic lymphocytic leukemia BCLL), hairy cell leukemia and chronic myelogenous leukemia) and myeloma (e.g. multiple myeloma). Additional B cell malignancies include small lymphocytic lymphoma, B cell prolymphocytic leukemia, lymphoplasmacytic lymphoma, splenic marginal zone lymphoma, plasma cell myeloma, solitary plasmacytoma of bone, extraosseous plasmacytoma, extra-nodal marginal zone B cell lymphoma of mucosa-associated (MALT) lymphoid tissue, nodal marginal zone B cell lymphoma, follicular lymphoma, mantle cell lymphoma, diffuse large B cell lymphoma, mediastinal (thymic) large B cell lymphoma, intravascular large B cell lymphoma, primary effusion lymphoma, Burkitt's lymphoma/leukemia, grey zone lymphoma, B cell proliferations of uncertain malignant potential, lymphomatoid granulomatosis, and post-transplant lymphoproliferative disorder.

The CD37 antibody may be administered alone or in combination with adjuvants that enhance the stability, facilitate administration of pharmaceutic compositions containing them in certain embodiments, provide increased dissolution or dispersion, increase activity, provide adjunct therapy, and the like. Advantageously, such combinations may utilize lower dosages of the active ingredient, thus reducing possible toxicity and adverse side effects.

Depending on the disorder to be treated, the CD37 antibody molecule, especially A2 and B2, may be used on its own or in combination with one or more additional therapeutic agents, in particular selected from DNA damaging or tubulin binding agents or therapeutically active compounds that inhibit angiogenesis, signal transduction pathways or mitotic checkpoints in cancer cells.

The additional therapeutic agent may be administered simultaneously with, optionally as a component of the same pharmaceutical preparation, or before or after administration of the CD37 antibody molecule, especially A2 and B2.

### DESCRIPTION OF THE FIGURES

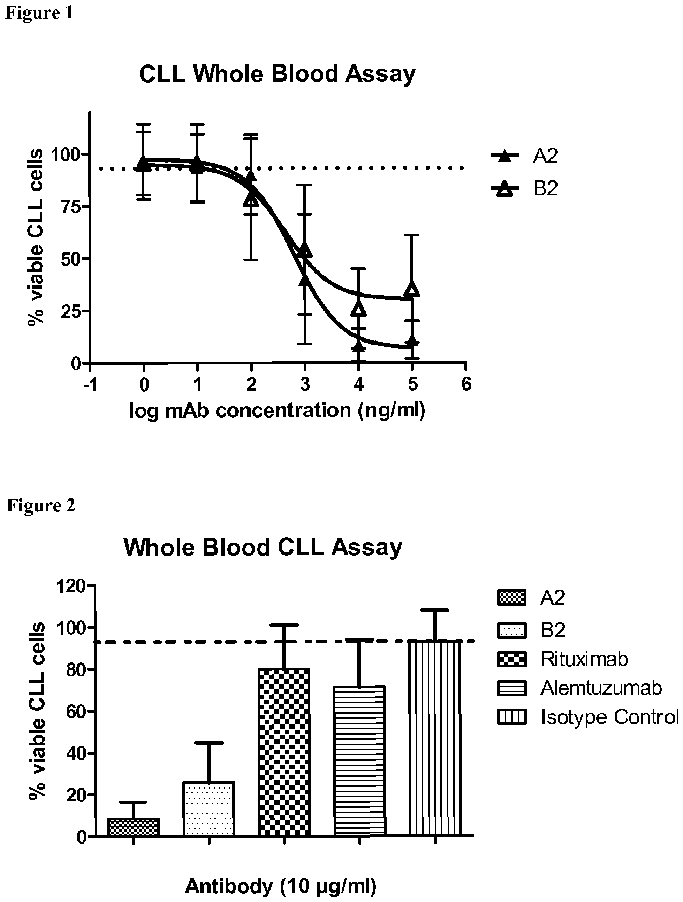

FIGURE 1: WHOLE BLOOD ASSAY: EFFECT OF ANTIBODIES A2 AND B2 ON VIABLE CLL CELLS.
   Whole blood samples from CLL patients were incubated with increasing concentrations (0.001µg/ml to 100µg/ml) of antibodies A2 and B2 for 3 hours. The percentage of viable CLL cells at the end of the incubation period in relation to antibody concentration is depicted, curves represent mean of 11 individual patient samples. Standard deviation for each concentration tested is indicated. A2: filled triangle; B2: open triangle. Dotted line represents activity of an isotype matched control antibody.
FIGURE 2: WHOLE BLOOD ASSAY WITH CLL PATIENT SAMPLES: COMPARISON OF DIFFERENT ANTIBODIES.
   Blood samples were incubated with 10 µg/ml of antibodies A2, B2, rituximab, alemtuzumab and an isotype matched control antibody for 3 hours. The percentage of viable CLL cells at the end of the incubation period is depicted, bars represent mean of 11 patient samples. Standard deviation is indicated. Dotted line represents activity of the isotype matched control antibody.

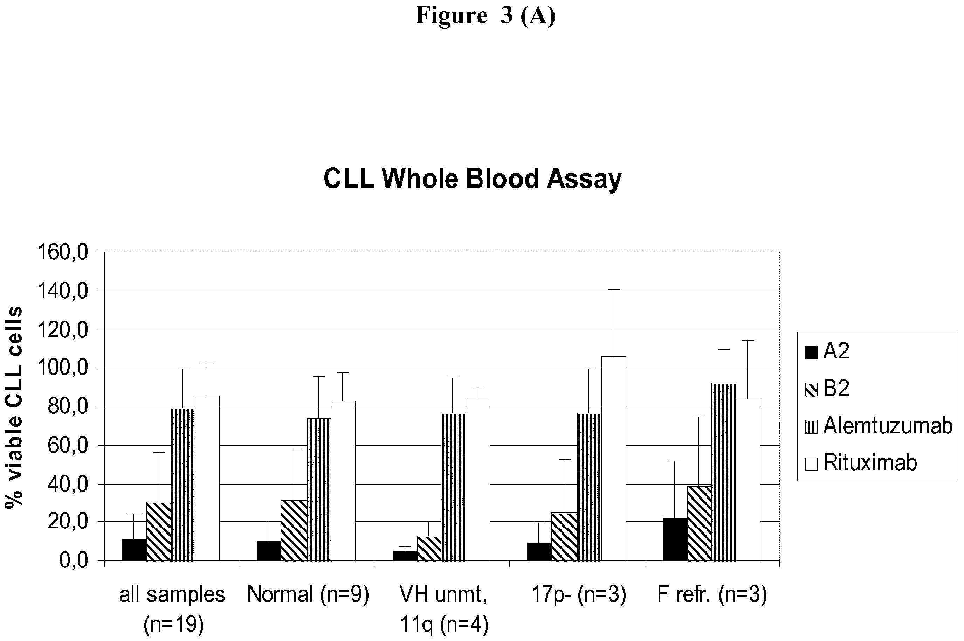

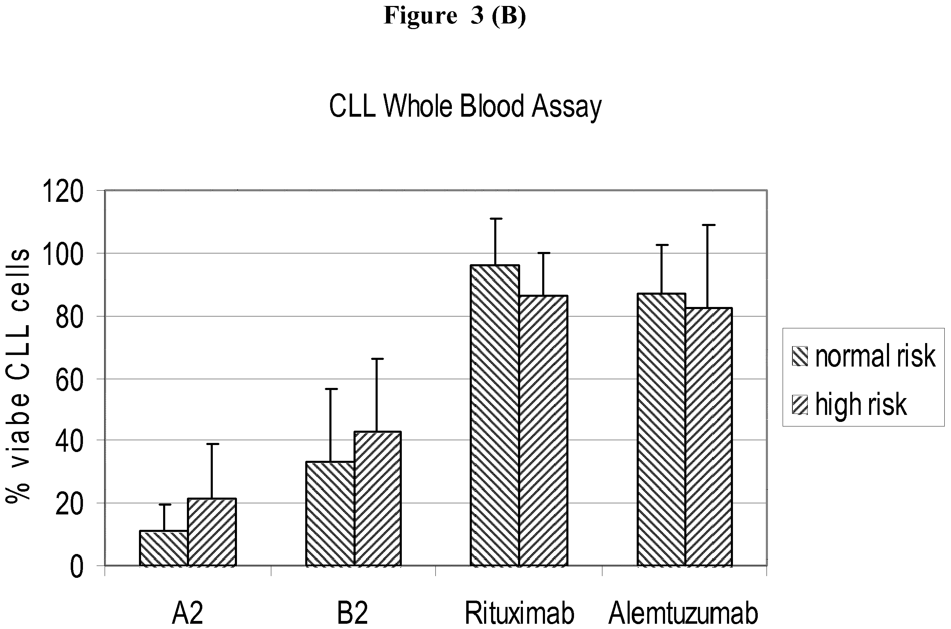

FIGURE 3: WHOLE BLOOD ASSAY WITH CLL PATIENT SAMPLES: COMPARISON OF DIFFERENT PATIENT SUBGROUPS.
   (A) Blood samples are incubated with 10 µg/ml of antibodies A2, B2, rituximab and alemtuzumab for 3 hours. The percentage of viable CLL cells at the end of the incubation period for different patient subgroups is depicted, bars represent mean of the indicated number of patient samples. Standard deviation is indicated.
   (B) Blood samples from normal risk patients (n = 11) and high risk patients (n=10) were incubated with 10 µg/ml of antibodies A2, B2, rituximab and alemtuzumab for 8 hours. The percentage of viable CLL cells at the end of the incubation period for the two patient subgroups is depicted, bars represent mean values, standard deviation is indicated.

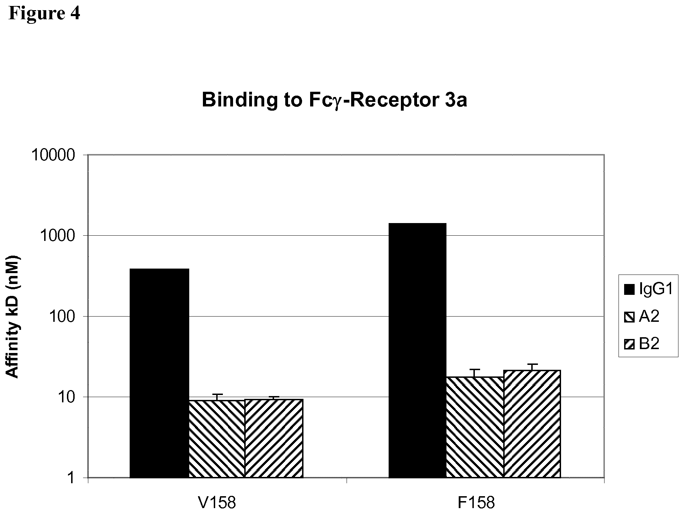

FIGURE 4: BINDING TO FCγ-RECEPTOR 3A
   The affinity of antibodies A2 and B2 to Fcγ-receptor 3a is determined by surface plasmon resonance analysis (SFP) (Edwards and Leatherbarrow, Analytical Biochemistry 246, 1997; Nieba et al., Analytical Biochemistry 234, 1996).
   Fcγ-receptor 3a protein high affinity allotype: V158.
   Fcγ-receptor 3a protein low affinity allotype: F158.
   A non Fc-engineered, IgG1-type of antibody is used as reference antibody.
   Antibodies are coated onto a sensor surface and the dissociation constant K_{D} of antibodies A2 and B2 is calculated using the rates of complex formation (kₐ) and dissociation (k_{d}) determined by SFP. Bars represent mean of 3 independent measurements, standard deviation is indicated.

### LEGEND TO SEQUENCE LISTING

SEQ ID NO 1: nucleic acid sequence variable heavy (Vh) chain
SEQ ID NO 2: amino acid sequence variable heavy chain
SEQ ID NO 3: nucleic acid sequence variable light (Vl) chain
SEQ ID NO 4: amino acid sequence variable light chain
SEQ ID NO 5: A2 heavy chain amino acid sequence
SEQ ID NO 6: A2 light chain amino acid sequence
SEQ ID NO 7: constant heavy chain amino acid sequence
SEQ ID NO 8: constant light chain amino acid sequence
SEQ ID NO 9: A4 heavy chain amino acid sequence
SEQ ID NO 10: A4 light chain amino acid sequence
SEQ ID NO 11: B2 heavy chain amino acid sequence
SEQ ID NO 12: B2 light chain amino acid sequence
SEQ ID NO 13: B4 heavy chain amino acid sequence
SEQ ID NO 14: B4 light chain amino acid sequence
SEQ ID NO 15: CDR1 heavy chain (H1)
SEQ ID NO 16: CDR2 heavy chain (H2)
SEQ ID NO 17: CDR3 heavy chain (H3)
SEQ ID NO 18: CDR1 light chain (L1)
SEQ ID NO 19: CDR2 light chain (L2)
SEQ ID NO 20: CDR3 light chain (L3)
SEQ ID NO 21: alternative CDR2 heavy chain (H2b)

### DETAILED DESCRIPTION OF THE INVENTION

Whole blood assays with CLL patient blood samples: In an initial experiment whole blood samples from 11 CLL patients are treated with increasing concentrations (0.001-100µg/ml) of the CD37 antibodies A2 and B2 for 3 hours. Both antibodies show effective and concentration-dependent CLL cell depletion in the whole blood assay. As shown in Figure 1, both A2 and B2 very potently deplete CLL cells with EC₅₀ values of about 1000 ng/ml. The CLL cell depleting effect of A2 and B2 at an antibody concentration of 10 µg/ml is compared to that of rituximab and alemtuzumab at the same antibody concentration after an incubation of the blood samples for 3 hours. As shown in Figure 2 CLL cell depletion of A2 (9% alive cells) and B2 (26% alive cells) at an antibody concentration of 10 µg/ml is clearly superior to that observed with alemtuzumab (71% alive cells) and rituximab (80% alive cells). Taken together these data clearly demonstrate that antibodies A2 and B2 exert a potent and concentration dependent depletion of CLL cells from whole blood samples *in vitro.* The degree of CLL cell depletion is clearly superior to that observed with rituximab and alemtuzumab.

In a specific embodiment of the present invention the effect of the CD37 antibodies A2 and B2 in cases with genomic aberrations (e.g. TP53 mutation or 17p13 deletion), in fludarabine refractory cases, and in patients with failure of previous anti-CD20 treatment is investigated (Figures 3A and 3B). These cases are generally considered as high risk or ultra-high risk patient populations with median survival of around 2 years with current treatment approaches. To this end blood samples from 21 patients are characterized, including 10 patients of the high risk or ultra-high risk group and 11 samples without any particular risk features (Figure 3B). We compare CLL cell depletion in the high risk group (17p13 deletion, TP53 mutation, fludarabine refractory) and the remaining patients without any particular risk. For both antibodies, A2 and B2, the CLL cell depletion is high in both risk groups, i.e. at 10 µg/ml A2 mean remaining CLL cells: 11.1% (8h) normal risk versus 21.3% high risk; 10 µg/ml B2 mean remaining CLL cells: 33.2% (8h) normal risk versus 43.1% high risk (Figure 3B).

In conclusion the CD37 antibodies, especially A2 and B2, show highly effective and potent CLL cell depletion in whole blood samples from CLL patients. This effect is high in all tested patient risk groups, especially blood samples from patients with high risk show a similar high degree of CLL cell depletion as blood samples from normal risk groups. Hence, CD37 antibodies, particularly A2 and B2, are highly effective in all tested CLL samples irrespective of genetic risk and are thus particularly well suited for the treatment of patients with increased risk of treatment failure, so called "high risk" patients or patient populations (e.g. TP53 mutation, 17p13 deletion, fludarabine refractory or failure of anti-CD20 therapy) or "ultra high risk" patients or patient populations.

### DEFINITIONS

The general embodiments "comprising" or "comprised" encompass the more specific embodiment "consisting of". Furthermore, singular and plural forms are not used in a limiting way. Terms used in the course of this present invention have the following meaning.

"CD37", a member of the tetraspanin superfamily, is a heavily glycosylated cell surface molecule with four transmembrane domains and two extracellular loops. CD37 is predominantly expressed on B cells and B cell malignancies, low level expression of CD37 has been reported on T cells, granulocytes, and monocytes. High levels of CD37 expression have been observed in samples of patients with chronic lymphocytic leukemia (CLL) and different subtypes of non-Hodgkin's lymphoma (NHL) including mantle cell lymphoma (MCL) (Schwartz-Albiez et al, Journal Immunol 140: 905-914, 1988; Barrena et al., Leukemia 19: 1376-1383, 2005). This expression pattern makes CD37 an attractive target for antibody-mediated cancer therapy.

Binding of a CD37-specific mAb to cancer cells may trigger various mechanisms of action: First, after the antibody binds to the extracellular domain of the CD37 antigen, it may activate the complement cascade and lyse the targeted cell. Second, an anti-CD37 antibody may mediate antibody-dependent cell-mediated cytotoxicity (ADCC) to the target cell, which occurs after the Fc portion of the bound antibody is recognized by appropriate receptors on cytotoxic cells of the immune system. Third, the antibody may alter the ability of B cells to respond to antigen or other stimuli. Finally, anti-CD37 antibody may initiate programmed cell death (apoptosis).

The terms "CD37 antibody", "CD37 antibody molecule", "anti-CD37 antibody" and "anti-CD37 antibody molecule" as used in the present invention specifically relate to an antibody with a binding specificity for CD37 antigen. Examples of such antibodies are known in the art and are further described below.
The terms "anti-CD37 antibody molecule", "anti-CD37 antibody", "CD37 antibody" and "CD37 antibody molecule" are used interchangeably.

The term "high risk patient", or "high risk" patient population means that the life expectancy of this patient group is very short (median of 2-3 years) and the response to current standard treatment is significantly worse than for patients without these high risk features. More specifically this group contains patients with fludarabine refractory disease which means patients who have received fludarabine alone or in combination and have either not responded (failure to achieve partial response or complete response) or have progressed within 6 months after treatment. Furthermore such high risk patients comprise "TP53 dysfunctional" patients and patients having chromosomal aberrations like 17p13 deletion.
"TP53 dysfunctional" patient means that patients have impaired function of the tumor suppressor gene TP53 or the p53 protein. This currently relates to inactivation of p53 protein by mutation or to deletion of one copy of the TP53 gene or a combination thereof. Either of these aberrations is associated with refractory CLL, poor response to standard treatment and short progression free survival and overall survival.
17p13 deletion means a deletion of the chromosome 17p13 or parts thereof by genetic events, e.g. genomic instability.
The term "ultra-high risk" patient or "ultra-high risk"patient population specifically comprises a group of high risk patients with particular poor prognosis or with a combination of risk factors, e.g. TP53 dysfunction and 17p13 deletion and refractory to fludarabine. Ultra-high risk patients may have a median life expectancy which is below that of high risk patients, e.g. median survival is less than 2 years. Furthermore ultra-high risk patients may be defined as patients who are initially refractory to treatment, e.g. fludarabine refractory.

The term "antibody" or "antibodies" comprises monoclonal, polyclonal, multispecific and single chain antibodies and fragments thereof such as for example Fab, Fab', F(ab')₂, Fc and Fc' fragments, light (L) and heavy (H) immunoglobulin chains and the constant, variable or hypervariable regions thereof as well as Fv and Fd fragments. The term "antibody" or "antibodies" comprises antibodies of human or non-human origin, humanised as well as chimeric antibodies and furthermore Fc-engineered antibodies or Fc-fusion molecules.
Fab fragments (fragment antigen binding = Fab) consist of the variable regions of both chains which are held together by the adjacent constant regions. They may be produced for example from conventional antibodies by treating with a protease such as papain or by DNA cloning. Other antibody fragments are F(ab')₂ fragments which can be produced by proteolytic digestion with pepsin.

By gene cloning it is also possible to prepare shortened antibody fragments which consist only of the variable regions of the heavy (VH) and light chain (VL). These are known as Fv fragments (fragment variable = fragment of the variable part). As covalent binding via the cysteine groups of the constant chains is not possible in these Fv fragments, they are often stabilised by some other method. For this purpose the variable regions of the heavy and light chains are often joined together by means of a short peptide fragment of about 10 to 30 amino acids, preferably 15 amino acids. This produces a single polypeptide chain in which VH and VL are joined together by a peptide linker. Such antibody fragments are also referred to as single chain Fv fragments (scFv). Examples of scFv antibodies are known in the art.

In past years various strategies have been developed for producing multimeric scFv derivatives. The intention is to produce recombinant antibodies with improved pharmacokinetic properties and increased binding avidity. In order to achieve the multimerisation of the scFv fragments they are produced as fusion proteins with multimerisation domains. The multimerisation domains may be, for example, the CH3 region of an IgG or helix structures ("coiled coil structures") such as the Leucine Zipper domains. In other strategies the interactions between the VH and VL regions of the scFv fragment are used for multimerisation (e.g. dia, tri- and pentabodies).

The term "diabody" is used in the art to denote a bivalent homodimeric scFv derivative. Shortening the peptide linker in the scFv molecule to 5 to 10 amino acids results in the formation of homodimers by superimposing VH/VL chains. The diabodies may additionally be stabilised by inserted disulphite bridges. Examples of diabodies can be found in the literature.

The term "minibody" is used in the art to denote a bivalent homodimeric scFv derivative. It consists of a fusion protein which contains the CH3 region of an immunoglobulin, preferably IgG, most preferably IgG1, as dimerisation region. This connects the scFv fragments by means of a hinge region, also of IgG, and a linker region. Examples of such minibodies are known in the art.

The term "triabody" is used in the art to denote a trivalent homotrimeric scFv derivative. The direct fusion of VH-VL without the use of a linker sequence leads to the formation of trimers.

The fragments known in the art as mini antibodies which have a bi, tri- or tetravalent structure are also derivatives of scFv fragments. The multimerisation is achieved by means of di-, tri- or tetrameric coiled coil structures.

There are also "scaffold proteins" or "scaffold antibodies" known in the art. Using this term, a scaffold protein means any functional domain of a protein, especially an antibody, that is coupled by genetic cloning or by co-translational processes with another protein or part of a protein that has another function.

The term "Complementary determining region" or "CDR" or "CDRs" of an antibody / antibody molecule means the hypervariable regions (also called Complementarity Determining Regions, abbreviated to "CDRs") of immunoglobulins. The CDRs were originally defined by Kabat et al., ("Sequences of Proteins of Immunological Interest" Kabat, E., of al., U.S. Department of Health and Human Services, (1983) and Kabat E. A., Wu T. T., Perry H. M., Gottesman K. S. and Foeller C. Sequences of Proteins of Immunological Interest (5th Ed.). NIH Publication No. 91-3242. U.S. Department of Health and Human Services, Public Health Service, National Institutes of Health, Bethesda, MD 1991) based on extent of sequence variability of numerous antibody sequences. The CDRs are believed to contact the target antigen of an antibody and to be primarily responsible for binding. Chothia et al (Chothia and Lesk, J. Mol. Biol., 196:901- 917 (1987)) have given an alternate definition of the hypervariable regions or CDRs. The Chothia definition is based on the residues that constitute the loops in the 3-dimensional structures of antibodies.

In the specific context of the present invention the CDRs are determined on the basis of the Kabat system. From the sequences of the variable regions as shown in SEQ ID NO:2 and SEQ ID NO:4, the CDR sequence can be routinely determined by searching the Kabat sequence database for sequence features. The 3 CDRs contained within the variable heavy chain as shown in SEQ ID NO:2 comprise preferably positions 31-35 (H1, SEQ ID NO: 15), 50-66 (H2, SEQ ID NO: 16) or 50-62 (H2b, SEQ ID NO: 21) and 99 - 105 (H3, SEQ ID NO: 17), the 3 CDRs contained within the variable light chain as shown in SEQ ID NO:4 comprise preferably positions 24-34 (L1, SEQ ID NO: 18), 50-56 (L2, SEQ ID NO: 19) and 89-97 (L3, SEQ ID NO: 20).

### EMBODIMENTS

The present invention concerns a CD37 antibody for the treatment of a high risk patient suffering from a B cell malignancy.
In another embodiment the present invention concerns a pharmaceutical composition comprising a CD37 antibody for the treatment of a high risk patient suffering from a B cell malignancy and a pharmaceutically acceptable excipient or carrier.

The invention further concerns the use of a CD37 antibody or a pharmaceutical composition comprising a CD37 antibody for the manufacture of a medicament for treatment of a high risk or ultra high risk patient suffering from a B cell malignancy.

The present invention concerns furthermore a CD37 antibody or a pharmaceutical composition comprising a CD37 antibody for use in the treatment of a high risk patient suffering from a B cell malignancy. The present invention concerns specifically a CD37 antibody or a pharmaceutical composition comprising a CD37 antibody for use in the treatment of chronic lymphocytic leukemia (CLL) high risk patient(s).

The present invention concerns further a CD37 antibody or a pharmaceutical composition comprising a CD37 antibody for use in a method for treatment of a high risk patient suffering from a B cell malignancy. The present invention concerns specifically a CD37 antibody or a pharmaceutical composition comprising a CD37 antibody for use in a method for treatment of chronic lymphocytic leukemia (CLL) high risk patient(s).

The invention further concerns a method for treating a B cell malignancy comprising administrating a therapeutically effective amount of a CD37 antibody or a pharmaceutical composition comprising a CD37 antibody to a high risk patient in need thereof.

The invention furthermore concerns a method for treating a high risk patient suffering from a B cell malignancy, the method comprising administering a therapeutically effective amount of a CD37 antibody to said patient.

In another embodiment, the invention concerns a method for treating a B cell malignancy or a high risk patient suffering from a B cell malignancy comprising (i) identifying a high risk or ultra-high risk patient in need of said treatment for B cell malignancy and (ii) administering a therapeutically effective amount of a CD37 antibody or a pharmaceutical composition comprising a CD37 antibody to said high risk or ultra-high risk patient.

In a specific embodiment of the above described invention the high risk patient is selected from a group consisting of: a patient refractory to fludarabine treatment, a patient with TP53 dysfunction, a patient with 17p13 deletion, and a patient no longer responding to rituximab treatment. In a specific embodiment, the high risk patient has a life expectancy of median 2-3 years, especially no more than 2 years.
In a specific embodiment of the present invention the patient is an ultra high risk patient.

In a specific embodiment the antibody is an antibody comprising: a) The CDRs contained within the variable heavy chain as shown in SEQ ID NO:2, and b) The CDRs contained within the variable light chain as shown in SEQ ID NO:4. Preferably said CDRs contained within the variable heavy chain have SEQ ID NOs: 15, 16 or 21, and 17. Preferably said CDRs contained within the variable light chain have SEQ ID NOs: 18, 19 and 20.

In a specific embodiment of the invention, the anti-CD37 antibody molecule/ the CD37 antibody is a chimeric antibody defined by
i) a variable heavy chain comprising the amino acid sequence shown in SEQ ID NO: 2;
ii) a variable light chain comprising the amino acid sequence shown in SEQ ID NO:4. Preferably the constant heavy and light chains are of human origin. The construction and production of chimeric mouse/human antibodies is well known in the art.

Preferably, i) the constant heavy chain is a IgG1 chain, and ii) the constant light chain is a kappa chain. In a specific embodiment the antibody molecule has one or more mutations in the Fc domain that modulate one or more effector functions, preferably said modulation of effector function is an increase in antibody-dependent cell-mediated cytotoxicity, preferably said one or more mutations in the Fc domain is a combination of substitutions at positions 239 and 332, or 236 and 332, or 236, 239 and 332, numbered according to the Kabat EU numbering index, preferably said substitutions are I332E and S239D or I332E and G236A, or S239D, I332E and G236A.

Preferably, the CD37 antibody has a heavy chain comprising the amino acid sequence of SEQ ID NO: 5 and preferably a light chain comprising the amino acid sequence of SEQ ID NO:6. Further preferred is the CD37 antibody having a heavy chain comprising the amino acid sequence of SEQ ID NO:7 and preferably a light chain comprising the amino acid sequence of SEQ ID NO:8. Also preferred is the CD37 antibody having a heavy chain comprising the amino acid sequence of SEQ ID NO:9 and preferably a light chain comprising the amino acid sequence of SEQ ID NO:10.

In another specific embodiment of the invention, the anti-CD37 antibody molecule binds to human CD37 and is derived from a murine monoclonal antibody that is defined by
i) a variable heavy chain comprising the amino acid sequence shown in SEQ ID NO: 2; and
ii) a variable light chain comprising the amino acid sequence shown in SEQ ID NO:4, wherein said antibody is a humanized antibody defined by
   a) CDRs contained within the variable heavy chain as shown in SEQ ID NO:2,
   b) CDRs contained within the variable light chain as shown in SEQ ID NO:4,
   c) frameworks supporting said CDRs that are derived from a human antibody,
wherein the constant heavy and light chains are from a human antibody. Preferably said CDRs contained within the variable heavy chain have SEQ ID NOs: 15, 16 or 21, and 17. Preferably said CDRs contained within the variable light chain have SEQ ID NOs: 18, 19 and 20.

In a specific embodiment the antibody molecule has one or more mutations in the Fc domain that modulate one or more effector functions, preferably said modulation of effector function is an increase in antibody-dependent cell-mediated cytotoxicity, preferably said one or more mutations in the Fc domain is a combination of substitutions at positions 239 and 332, or 236 and 332, or 236, 239 and 332, numbered according to the Kabat EU numbering index, preferably said substitutions are I332E and S239D or I332E and G236A, or S239D, I332E and G236A.

In a preferred embodiment the CD37 antibody has a heavy chain comprising the amino acid sequence of SEQ ID NO:11 and preferably a light chain comprising the amino acid sequence of SEQ ID NO:12. Further preferred is an antibody having a heavy chain comprising the amino acid sequence of SEQ ID NO:13 and preferably a light chain comprising the amino acid sequence of SEQ ID NO:14.

The Fc variants in the antibodies of the present invention are defined according to the amino acid modifications that compose them. Thus, for example, I332E is an Fc variant with the substitution I332E relative to the parent Fc polypeptide. Likewise, S239D/I332E defines an Fc variant with the substitutions S239D and I332E and S239D/I332E/G236A defines an Fc variant with the substitutions S239D, I332E, and G236A relative to the parent Fc polypeptide.
Numbering is according to the EU numbering scheme (Kabat et al., 1991, see above), which refers to the numbering of the EU antibody (Edelman et al. Proc Natl Acad Sci USA 63: 78-85, 1969). The person skilled in the art will appreciate that these conventions consist of nonsequential numbering in specific regions of an immunoglobulin sequence, enabling a normalized reference to conserved positions in immunoglobulin families.
In the above described antibodies, the substituted positions 236, 239 and 332 correspond to positions 119, 122 and 215, respectively, of the IgG1 heavy chain depicted in SEQ ID NO:7. In the full-length sequences of the heavy chains of antibodies A2, A4, B2 and B4 depicted in SEQ ID NOs: 5, 9, 11 and 13, the substituted amino acids are at positions 235, 238 and 331.

In a specific embodiment of the present invention, the B cell malignancy is selected from the group consisting of: B cell lymphomas, Hodgkin's disease, B cell non-Hodgkin's lymphoma (NHL), Waldenström's macroglobulinaemia (also called lymphoplasmacytic lymphoma or immunocytoma), central nervous system lymphomas, leukemias, acute lymphoblastic leukemia (ALL), chronic lymphocytic leukemia (CLL; also termed B cell chronic lymphocytic leukemia B-CLL), hairy cell leukemia, chronic myoblastic leukemia, myelomas, multiple myeloma, small lymphocytic lymphoma, B cell prolymphocytic leukemia, lymphoplasmacytic lymphoma, splenic marginal zone lymphoma, plasma cell myeloma, solitary plasmacytoma of bone, extraosseous plasmacytoma, extra-nodal marginal zone B cell lymphoma of mucosa-associated (MALT) lymphoid tissue, nodal marginal zone B cell lymphoma, follicular lymphoma, aggressive B-cell lymphoma, mantle cell lymphoma, diffuse large B cell lymphoma, mediastinal (thymic) large B cell lymphoma, intravascular large B cell lymphoma, primary effusion lymphoma, Burkitt's lymphoma/leukemia, grey zone lymphoma, B cell proliferations of uncertain malignant potential, lymphomatoid granulomatosis, and post-transplant lymphoproliferative disorder. Preferably said B cell malignancy is CLL.

The invention further concerns a method of depleting CD37 expressing B cells from a population of TP53 deficient cells comprising administering to said population of cells a CD37 antibody or a pharmaceutical composition comprising a CD37 antibody and a pharmaceutically acceptable excipient or carrier. Preferably, said method is carried out *in vitro.*
In a specific embodiment said CD37 antibody of said method is an antibody comprising: a) The CDRs contained within the variable heavy chain as shown in SEQ ID NO:2, and b) The CDRs contained within the variable light chain as shown in SEQ ID NO:4. Specific examples of such CD37 antibodies, which are useful for said method of depletion, are described above.

The practice of the present invention will employ, unless otherwise indicated, conventional techniques of cell biology, molecular biology, cell culture, immunology and the like which are in the skill of one in the art. These techniques are fully disclosed in the current literature.
All publications and patent applications mentioned in this specification are indicative of the level of skill of those skilled in the art to which this invention pertains. All publications and patent applications cited herein are hereby incorporated by reference in their entirety in order to more fully describe the state of the art to which this invention pertains. The invention generally described above will be more readily understood by reference to the following examples, which are hereby included merely for the purpose of illustration of certain embodiments of the present invention. The following examples are not limiting. They merely show possible embodiments of the invention. A person skilled in the art could easily adjust the conditions to apply it to other embodiments.

### EXPERIMENTAL

### MATERIALS AND METHODS

The CLL cell depleting activity of several antibodies (A2, B2, rituximab, alemtuzumab) is studied in a set of CLL patient blood samples *in vitro.* CLL samples are characterized with respect to genetics (genomic aberrations, TP53 mutation, 17p13 deletion), as well as clinical course and immunophenotype. TP53 mutation status can be determined by sequencing of the coding exons of the DNA binding domain of p53 (e.g. exons 4-10) or other relevant exons by automated fluorescent sequencing as described by Zenz et al. (Zenz T, Kröber A, Scherer K, Häbe S, Bühler A, Benner A, Denzel T, Winkler D, Edelmann J, Schwänen C, Döhner H, Stilgenbauer S. Monoallelic TP53 inactivation is associated with poor prognosis in chronic lymphocytic leukemia: results from a detailed genetic characterization with long-term follow-up. Blood. 2008 Oct 15;112(8):3322-9. Epub 2008 Aug 8.) or by other suitable methods known in the art (for example as described in Coll-Mulet et al., Multiplex ligation-dependent probe amplification for detection of genomic alterations in chronic lymphocytic leukaemia., Br J Haematol. 2008 Sep;142(5):793-801. Epub 2008 Jun 17.). Analysis of genomic aberrations (e.g. deletion of chromosome 17p13) and VH status (e.g. IGVH mutation status) can be performed as described in the literature (e.g. Döhner et al., Genomic aberrations and survival in chronic lymphocytic leukemia. N Engl J Med. 2000 Dec 28;343(26):1910-6; Kröber et al., Additional genetic high-risk features such as 11q deletion, 17p deletion, and V3-21 usage characterize discordance of ZAP-70 and VH mutation status in chronic lymphocytic leukemia. J Clin Oncol. 2006 Feb 20;24(6):969-75. Epub 2006 Jan 17.). Fluorescence *in situ* hybridization (FISH) is a widely used method known in the art for the detection of genomic aberrations and deletion of chromosomes, e.g. 17p13 deletion (Döhner et al., 11q deletions identify a new subset of B-cell chronic lymphocytic leukemia characterized by extensive nodal involvement and inferior prognosis. Blood. 1997 Apr 1;89(7):2516-22.).

To study the effect on CLL cells, we assess the CD37 antibodies in parallel with rituximab and alemtuzumab after *in vitro* treatment in a whole blood culture system. 20 µl of antibody diluted in PBS are added to 180 µl of heparinised or ACD (acid-citrate-dextrose) blood and incubated for 3h or 8h at room temperature in an 48-well plate under constant shaking. Thereafter an aliquot of the blood - antibody mixture is transferred into BD TruCount tubes and 20 µl of a FITC-labelled CD19 antibody is added for identification of CLL cells. After lysis of erythrocytes the samples are measured with a FACS Calibur flow cytometry device. Viable CLL cells are determined via their CD 19 positivity and appearance in side scatter. Quantification of viable CLL cells is performed by using fluorescently labelled beads as internal standard which are included in known numbers in the TruCount tubes. Concentration response curves are calculated using the GraphPad Prism software package version 5.02.

### EXAMPLES

### EXAMPLE 1: CLL WHOLE BLOOD ASSAY

The effect of antibody treatment on CLL cells in whole blood samples derived from CLL patients is assessed with a whole blood assay. Increasing concentrations (0.001µg/ml to 100µg/ml) of antibodies A2 and B2 are added to heparinised or acid-citrate-dextrose blood and incubated for 3 hours at room temperature. After antibody incubation the remaining viable CLL cells (CD19-positive) are determined by FACS analysis using a quantitative assay. As shown in Figure 1, both A2 and B2 very potently deplete CLL cells with EC₅₀ values of about 1000 ng/ml. The average maximum degree of CLL cell depletion at high antibody concentrations is more than 90% for A2 and about 75% for B2. These data demonstrate that antibodies A2 and B2 exert a potent and concentration dependent depletion of CLL cells from whole blood samples of CLL patients *in vitro.*

### EXAMPLE 2: CLL WHOLE BLOOD ASSAY: COMPARISON TO RITUXIMAB AND ALEMTUZUMAB

In order to compare the effects of A2 and B2 to approved antibodies for CLL treatment the CD20 antibody rituximab and the CD52 antibody alemtuzumab are tested in parallel in the same patient samples. As shown in Figure 2 CLL cell depletion at an antibody concentration of 10 µg/ml after 3 hours incubation with A2 (9% alive cells) and B2 (26% alive cells) is clearly superior to that observed with alemtuzumab (71% alive cells) and rituximab (80% alive cells). These data demonstrate that the degree of CLL cell depletion of A2 and B2 in whole blood samples from CLL patients is clearly superior to that observed with rituximab and alemtuzumab.

### EXAMPLE 3: CLL WHOLE BLOOD ASSAY: COMPARISON OF DIFFERENT PATIENT RISK GROUPS

We are particularly interested in the effect of A2 and B2 in cases with genomic aberrations (e.g. TP53 mutation or 17p13 deletion), fludarabine refractory cases and patients after failure of anti-CD20 therapy. These cases are generally considered as "high risk" or "ultra-high risk" patient populations. To this end blood samples from 19 (Figure 3A) to 21 (Figure 3B) patients are characterized, including 10 patients of the high risk or ultra-high risk group and 9 (Figure 3A) to 11 (Figure 3B) samples without any particular risk features. The high risk or ultra-high risk of patients group comprises patients with 17p13 deletion, TP53 mutation, patients refractory to fludarabine treatment and patient after anti-CD20 treatment. We compare CLL cell depletion in the high risk or ultra-high risk group (17p13 deletion, TP53 mutation, fludarabine refractory, after anti-CD20 treatment; n=10 Figure 3B) and the remaining patients without any particular risk (n=9 Figure 3A and n=11 Figure 3B). For both antibodies A2 and B2 the CLL cell depletion is high in both risk groups, i.e. at 10 µg/ml A2 mean remaining CLL cells: 11.1% (8h) normal risk; 21.3% high risk; 10 µg/ml B2 mean remaining CLL cells: 33.2% (8h) normal risk; 43.1% high risk (Figure 3B). As shown in Figure 3, especially Figure 3B, in all investigated patient subgroups the efficacy of A2 and B2 is clearly superior to that of rituximab and alemtuzumab. The findings from this analysis demonstrate that antibodies A2 and B2 show superior CLL depleting activity in all patients subgroups investigated and are especially suited to treat CLL patients with increased risk of treatment failure.

### EXAMPLE 4: ANTIBODIES A2 AND B2 SHOW HIGHLY IMPROVED BINDING TO FC GAMMA RECEPTOR 3A

The affinity of antibodies A2 and B2 to Fcγ-receptor 3a is determined by surface plasmon resonance analysis (SFP) (Edwards and Leatherbarrow, Determination of Association Rate Constants by an Optical Biosensor Using Initial Rate Analysis. Analytical Biochemistry 246, 1-6, 1997; Nieba et al., Competition BIAcore for Measuring True Affinities: Large Differences from Values Determined from Binding Kinetics. Analytical Biochemistry 234, 155-165, 1996). In brief, recombinant Fcγ-receptor 3a protein, either the high affinity V158 allotype or the low affinity F158 allotype, are coated onto a sensor surface and the dissociation constant K_{D} of antibodies A2 and B2 was calculated using the rates of complex formation (kₐ) and dissociation (k_{d}) determined by SFP. A non Fc-engineered, IgG1-type of antibody is used as reference antibody. A2 displayed a K_{D} of 8.8 nM to the V158 allotype and a K_{D} of 17.5 nM to the F158 allotype, similar as B2 which displays a K_{D} of 9.3 and 21.1 nM, respectively. In contrast, a non Fc-engineered IgG1 reference antibody displays a K_{D} of 379 nM to the high affinity allotype V158 and 1400 nM to the low affinity allotype F158. In summary, antibodies A2 and B2 display an about 40-fold increased binding to Fcγ-receptor 3a V158 and an about 60-fold to 80-fold increased binding to Fcγ-receptor 3a F158, which is expected to translate into highly increased antibody dependent cellular cytotoxicity (ADCC) and improved clinical efficacy of A2 and B2.

## Claims

1. A CD37 antibody for the treatment of a high risk patient suffering from a B cell malignancy.

2. A CD37 antibody or a pharmaceutical composition comprising a CD37 antibody for use in the treatment of a high risk patient suffering from a B cell malignancy, preferably for use in the treatment of chronic lymphocytic leukemia (CLL) high risk patient.

3. A CD37 antibody or a pharmaceutical composition comprising a CD37 antibody for use in a method for treatment of a high risk patient suffering from a B cell malignancy, preferably for use in a method for treatment of chronic lymphocytic leukemia (CLL) high risk patient.

4. The CD37 antibody according to claims 1-3, wherein the high risk patient is selected from a group consisting of: a patient refractory to fludarabine treatment, a patient with TP53 dysfunction, a patient with 17p13 deletion, and a patient no longer responding to rituximab treatment.

5. The CD37 antibody according to claims 1 to 4, wherein the high risk patient has a life expectancy of median 2-3 years, specifically no more than 2 years.

6. The CD37 antibody according to claims 1 to 5, whereby the antibody is an antibody comprising:
a. The CDRs contained within the variable heavy chain as shown in SEQ ID NO:2, preferably said CDRs have SEQ ID NOs: 15, 16 or 21, and 17, and
b. The CDRs contained within the variable light chain as shown in SEQ ID NO:4, preferably said CDRs have SEQ ID NOs: 18, 19 and 20.

7. The CD37 antibody according to claims 1 to 6, whereby the CD37 antibody is a chimeric antibody defined by
a. a variable heavy chain comprising the amino acid sequence shown in SEQ ID NO: 2, and
b. a variable light chain comprising the amino acid sequence shown in SEQ ID NO:4,
whereby the constant heavy and light chains are preferably of human origin.

8. The CD37 antibody according to claim 6, wherein said antibody is a humanized antibody defined by frameworks supporting said CDRs that are derived from a human antibody, and wherein the constant heavy and light chains are from a human antibody.

9. The CD37 antibody according to claim 6, whereby the antibody has a heavy chain comprising the amino acid sequence of SEQ ID NO:5 and preferably a light chain comprising the amino acid sequence of SEQ ID NO:6.

10. The CD37 antibody according to claim 6, whereby the antibody has a heavy chain comprising the amino acid sequence of SEQ ID NO: 7 and preferably a light chain comprising the amino acid sequence of SEQ ID NO: 8.

11. The CD37 antibody according to claim 6, whereby the antibody has a heavy chain comprising the amino acid sequence of SEQ ID NO:9 and preferably a light chain comprising the amino acid sequence of SEQ ID NO:10.

12. The CD37 antibody according to claim 6, whereby the antibody has a heavy chain comprising the amino acid sequence of SEQ ID NO:11 and preferably a light chain comprising the amino acid sequence of SEQ ID NO:12.

13. The CD37 antibody according to claim 6, whereby the antibody has a heavy chain comprising the amino acid sequence of SEQ ID NO:13 and preferably a light chain comprising the amino acid sequence of SEQ ID NO:14.

14. The CD37 antibody according to claims 1 to 13, whereby the B cell malignancy is selected from the group consisting of: B cell lymphomas, agressive B-cell lymphoma, Hodgkin's disease, B cell non-Hodgkin's lymphoma (NHL), lymphomas, Waldenström's macroglobulinaemia (also called lymphoplasmacytic lymphoma or immunocytoma), central nervous system lymphomas, leukemias, acute lymphoblastic leukemia (ALL), chronic lymphocytic leukemia (CLL; also termed B cell chronic lymphocytic leukemia BCLL), hairy cell leukemia, chronic myoblastic leukemia), myelomas, multiple myeloma), small lymphocytic lymphoma, B cell prolymphocytic leukemia, lymphoplasmacytic lymphoma, splenic marginal zone lymphoma, plasma cell myeloma, solitary plasmacytoma of bone, extraosseous plasmacytoma, extra-nodal marginal zone B cell lymphoma of mucosa-associated (MALT) lymphoid tissue, nodal marginal zone B cell lymphoma, follicular lymphoma, mantle cell lymphoma, diffuse large B cell lymphoma, mediastinal (thymic) large B cell lymphoma, intravascular large B cell lymphoma, primary effusion lymphoma, Burkitt's lymphoma/leukemia, grey zone lymphoma, B cell proliferations of uncertain malignant potential, lymphomatoid granulomatosis, and post-transplant lymphoproliferative disorder, whereby the B cell malignancy is preferably CLL.

15. A method for treating a B cell malignancy comprising administrating a therapeutically effective amount of
a. a CD37 antibody according to claims 1 to 14 or
b. a pharmaceutical composition comprising a CD37 antibody to a high risk or ultra high risk patient in need thereof.

16. The method according to claim 15, whereby the antibody is an antibody comprising:
a. The CDRs contained within the variable heavy chain as shown in SEQ ID NO:2, preferably said CDRs have SEQ ID NOs: 15, 16 or 21, and 17, and
b. The CDRs contained within the variable light chain as shown in SEQ ID NO:4, preferably said CDRs have SEQ ID NOs: 18, 19 and 20.

17. The method according to claim 15 or 16, whereby the CD37 antibody is a chimeric antibody defined by
a. a variable heavy chain comprising the amino acid sequence shown in SEQ ID NO: 2, and
b. a variable light chain comprising the amino acid sequence shown in SEQ ID NO:4,
whereby the constant heavy and light chains are preferably of human origin.

18. The method according to claim 16, wherein said antibody is a humanized antibody defined by frameworks supporting said CDRs that are derived from a human antibody, and wherein the constant heavy and light chains are from a human antibody.

19. The method according to claim 16, whereby the antibody has a heavy chain comprising the amino acid sequence of SEQ ID NO:5 and preferably a light chain comprising the amino acid sequence of SEQ ID NO:6.

20. The method according to claim 16, whereby the antibody has a heavy chain comprising the amino acid sequence of SEQ ID NO: 7 and preferably a light chain comprising the amino acid sequence of SEQ ID NO: 8.

21. The method according to claim 16, whereby the antibody has a heavy chain comprising the amino acid sequence of SEQ ID NO:9 and preferably a light chain comprising the amino acid sequence of SEQ ID NO:10.

22. The method according to claim 16, whereby the antibody has a heavy chain comprising the amino acid sequence of SEQ ID NO:11 and preferably a light chain comprising the amino acid sequence of SEQ ID NO: 12.

23. The method according to claim 16, whereby the antibody has a heavy chain comprising the amino acid sequence of SEQ ID NO:13 and preferably a light chain comprising the amino acid sequence of SEQ ID NO:14.

24. The method of claims 15 to 23, whereby the B cell malignancy is selected from the group consisting of: B cell lymphomas, agressive B-cell lymphoma, Hodgkin's disease, B cell non-Hodgkin's lymphoma (NHL), lymphomas, Waldenström's macroglobulinaemia (also called lymphoplasmacytic lymphoma or immunocytoma), central nervous system lymphomas, leukemias, acute lymphoblastic leukemia (ALL), chronic lymphocytic leukemia (CLL; also termed B cell chronic lymphocytic leukemia BCLL), hairy cell leukemia, chronic myoblastic leukemia), myelomas, multiple myeloma), small lymphocytic lymphoma, B cell prolymphocytic leukemia, lymphoplasmacytic lymphoma, splenic marginal zone lymphoma, plasma cell myeloma, solitary plasmacytoma of bone, extraosseous plasmacytoma, extra-nodal marginal zone B cell lymphoma of mucosa-associated (MALT) lymphoid tissue, nodal marginal zone B cell lymphoma, follicular lymphoma, mantle cell lymphoma, diffuse large B cell lymphoma, mediastinal (thymic) large B cell lymphoma, intravascular large B cell lymphoma, primary effusion lymphoma, Burkitt's lymphoma/leukemia, grey zone lymphoma, B cell proliferations of uncertain malignant potential, lymphomatoid granulomatosis, and post-transplant lymphoproliferative disorder, whereby the B cell malignancy is preferably CLL.

25. The method of claims 15 to 24, wherein the high risk patient is selected from a group consisting of: a patient refractory to fludarabine treatment, a patient with TP53 dysfunction, a patient with 17p13 deletion, and a patient no longer responding to rituximab treatment.

26. The method of claims 15 to 25, wherein the high risk patient has a life expectancy of median 2-3 years, specifically no more than 2 years.

27. A method of depleting CD37 expressing B cells from a population of TP53 deficient cells comprising administering to said population of cells a CD37 antibody or a pharmaceutical composition comprising a CD37 antibody and a pharmaceutically acceptable excipient or carrier, wherein said method is preferably carried out *in vitro.*
